(19) Europäisches Patentamt
European Patent Office
Office européen des brevets

(11) Numéro de publication: 0 275 270 B1

(12) **FASCICULE DE BREVET EUROPÉEN**

(45) Date de publication du fascicule du brevet: 21.03.90

(51) Int. Cl. [5]: C 07 B 59/00

(21) Numéro de dépôt: 87904273.7

(22) Date de dépôt: 01.07.87

(86) Numéro de dépôt international: PCT/FR 87/00256

(87) Numéro de publication international: WO 88/00175 (14.01.88 Gazette 88/02)

(54) **PROCEDE DE RADIOIODATION DE GANGLIOSIDES PAR VOIE ENZYMATIQUE.**

(30) Priorité: 03.07.86 FR 8609668

(43) Date de publication de la demande: 27.07.88 Bulletin 88/30

(45) Mention de la délivrance du brevet: 21.03.90 Bulletin 90/12

(84) Etats contractants désignés: AT BE CH DE FR GB IT LI LU NL SE

(56) Documents cité:

Cellular Immunology, vol. 5, no. 3, 1972 I. Schenkein et al.: "The use of glucose oxidase as a generator of H2 O2 in the enzymatic radioiodination of components of cell surfaces", pp 490-493 (voir le document en entier)

Journal of Immunological Methods, vol.59, no. 1, 1983, Elsevier Science Publishers; W.Cushley et al.: "On the fidelity of the lactoperoxidase method of cell membrane radioiodination: an electron microspocic autoradiographic study", pp 1-11 (voir le document en entier)

Pharmacological Research Communications, vol.17, no. 10, 1985, The Italian Pharmacological Society. M. Zalutsky et al.: "Radioiodinated ganglioside G M1: A potential tool for the investigation of ganglioside function In Vivo", pp 897-912 (voir le document en entier) .

(73) Titulaire: CENTRE NATIONAL DE LA RECHERCHE SCIENTIFIQUE
15, quai Anatole France
F-75007 Paris (FR)

(72) Inventeur: VINCENDON, Guy
16, avenue du Général de Gaulle
F-67000 Strasbourg (FR)
Inventeur: MERSEL, Marcel
4, rue de Stockholm
F-67000 Strasbourg (FR)

(74) Mandataire: Ahner, Francis
CABINET REGIMBEAU 26, avenue Kléber
F-75116 Paris (FR)

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe dipposition (Art. 99(1) Convention sur le brevet européen).

EP 0 275 270 B1

## Description

La présente invention concerne un procédé de marquage de gangliosides par de l'iode radioactif par voie enzymatique.

Dans le système nerveux central, la membrane plasmique de la cellule neuronale est particulièrement enrichie en gangliosides définis comme des glycosphingolipides contenant de l'acide sialique, principalement l'acide N-acétyl neuraminique.

On indiquera ci-après la structure des cinq gangliosides majeurs présents dans le cerveau des mammifères, à savoir GM1, GD1a, GD1b, GT1b, GQ1b:

Galactose N-Acétyl galactosamine Galactose Glucose

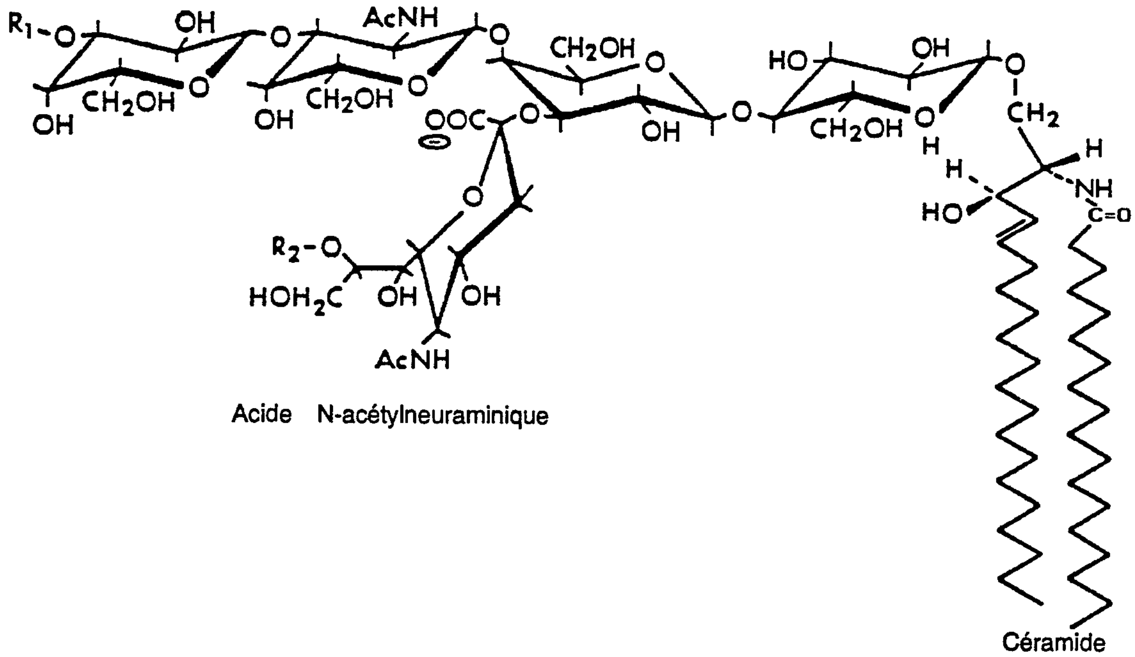

Acide N-acétylneuraminique

Céramide

GM1, $R_1 = R_2 = H$;
GD1a, $R_1$ = NeuAc, $R_2 = H$;
GD1b, $R_1 = H$, $R_2$ = NeuAc;
GT1b, $R_1 = R_2$ = NeuAc;
GQ1b, $R_1$ = NeuAc (2 - 8) NeuAc, $R_2$ = NeuAc.

La présente invention est destinée à effectuer la radioiodation d'un ou plusieurs gangliosides choisi(s) parmi GM3, GM2, GM1, GD3, GD1a, GD1b, GT1b, Gq, ainsi que leurs mélanges.

De nombreuses recherches, qui avaient pour but de déterminer le rôle physiologique des gangliosides dans le système nerveux, ont abouti aux hypothèses suivantes:

- Les gangliosides seraient impliqués dans la maturation et la différenciation du neurone en agissant comme des agents neurotropes ou neuritogènes (SPOERRI, P.E. (1983) Int. J. Devl. Neuroscience 1, 383 et LESKAWA, K.C. and HOGAN, E.L. (1985) J. Neuroscience Res.
- Les gangliosides moduleraient les activités d'enzymes clés (JANIGRO, D., DI GREGORIO, F., VYSKOCIL, F. and GORIO, A. (1984) J. Neuroscience Res. 12, 499) ou les propriétés de certains récepteurs (BRENNER E.G., HAKOMORI, S.I., BOWEN-POPE, D.F. RAINES, E. and ROSS, R. (1984) J. Biol. Chem. 259, 6818) de la membrane plasmique.
- Les gangliosides agiraient comme des "agents réparateurs", suite à des lésions cérabrales (WOJCIK, M., ULAS, J., ODERFELD-NOWAK, B. (1982) Neuroscience 7, 495).

Afin de mieux suivre l'insertion et le métabolisme des gangliosides dans la cellules nerveuse, on a déjà proposé d'utiliser des précurseurs radioactifs ($^{3}H$,$^{14}C$, rayonnement β) (TETTAMANTI, G., GHIDONI, R., VENERANDO, B., FIORILLI, A. and SANDRO, S., Colloque INSERM-CNRS, Aspects cellulaires et pathologiques du métabolisme des glycoconjugués, INSERM 1984 (DREYFUS, H., MASSARELI, R., FREYSZ, L. et REBEL, G., eds) 126, 135) et (YAVIN, E. RICHTER-LANSBERG, C., DUKSIN, D. and) YAVIN, Z. Proc. Natl. Acad. Sci. USA (1984) 81, 5638), pour marquer une partie de la molécule ou encore de synthétiser des gangliosides portant un marqueur de spin (radical nitroxyde) (SCHWARZMANN, G., SONDERFELD, S., CONZELMANN, E., MARSH, D. and SANDHOFF, K. Colloque INSERM-CNRS, Aspects cellulaires et pathologiques du métabolisme des glycoconjugués, INSERM 1984 (DREYFUS, M., MASSARELI, R., FREYSZ, L. et REBEL, G., eds) 126, 195).

La présente invention a eu pour but d'insérer un élément radioactif, en particulier de l'iode 125, dans les gangliosides et de préférence dans le domaine hydrophobe composant les gangliosides majeurs.

L'invention propose donc un nouveau procédé enzymatique permettant d'obtenir des gangliosides marqués par de l'iode radioactif en utilisant une peroxydase et un générateur de peroxyde. Ce procédé peut être mis en oeuvre avantageusement en milieu aqueux, dans des conditions réactionnelles douces et en exerçant un contrôle de la génération de peroxyde.

On rappellera que, dans l'état de la technique, aucune méthode chimique ou enzymatique permettant le marquage avec des éléments à haute énergie (rayonnement γ) des différentes espèces moléculaires des gangliosides n'a été décrite.

Des réactions d'halogénation catalysées par des haloperoxydases ont été décrites par d'autres auteurs (LEE, T.D., GEIGERT, J., DALIETOS, D.J. and HIRANO, D.S. (1983) Biochem. Biophys. Res. Commun. 110, 880; GEIGERT, J., NEIDLEMAN, S.L. and DALIETOS, D.J. (1983) J. Biol. Chem. 258, 2273; NEIDLEMAN, S.L. and GEIGERT, J. (1983) Trends in Biotechnol. 1, 21 et GEIGERT, J., and NEIDLEMAN, S.L. (1983) Devel. in Industrial Microbiology 24, 385). Mais les substrats sont des alcènes ou alcynes, ne dépassant pas 8 carbones, la réaction se fait en milieu acide (pH 3), l'eau oxygénée n'est pas générée par un système enzymatique et l'halogénation se fait uniquement par addition sur double ou triple liaison et non par substitution.

En revanche, la radioiodation selon l'invention se déroule dans des conditions salines et de pH physiologiques et aucune modification de la structure (à part l'halogénation) de la molécule ne peut intervenir. Aussi le marquage se fait sur des chaînes aliphatiques contenant plus de 8 carbones et donc sur des structures micellaires.

La synthèse ou biosynthèse de gangliosides marqués par l'isotope ($^{14}C$) ou par l'isotope ($^{3}H$), et ceci même dans des positions spécifiques, présentent les inconvénients suivants:

- Le temps nécessaire afin d'obtenir des gangliosides radioactifs ($^{14}C$,$^{3}H$) est de l'ordre de 2 jours dans les conditions les plus optimales. Afin d'augmenter le rendement, une synthèse chimique à grande échelle est nécessaire, et la durée de l'opération est d'autant plus longue.
- L'utilisation de gangliosides tritiés est désavantageuse, le phénomène d'échange isotopique in vitro et in vivo risque de perturber l'interprétation des résultats finaux. La synthèse d'un ganglioside tritié sur une position spécifique de la molécule augmenterait de beaucoup le coût de la production.
- Les composés marqués au carbone (C) ou au tritium ($^{3}H$) nécessitent l'addition de solvants organiques (scintillateurs), afin de pouvoir quantifier la radioactivité (cpm:coups par minute). Après le comptage de la radioactivité ces produits sont donc irrécupérables. De plus, le ($^{14}C$) et le ($^{3}H$) émettent des rayonnements β et, afin de pouvoir détecter les composés radioactifs, une activité spécifique élevée est nécessaire.

Les gangliosides marqués conformément au procédé de l'invention sont utiles en recherche fondamentale tant in vivo que in vitro, ainsi qu'en tant que matériel de test ou de diagnostic.

Le procédé de l'invention est caractérisé en ce que l'on met à réagir une solution aqueuse d'au moins un ganglioside avec un composé iodé radioactif, en particulier un iodure $^{125}I^-$, en présence d'une peroxydase catalysant les réactions d'halogénation et d'un peroxyde ou générateur de peroxyde, puis après achèvement de la réaction, en ce que l'on purifie et l'on récupère les gangliosides radiomarqués.

Selon une caractéristique particulière du procédé de l'invention, ladite peroxydase est choisie parmi la lactoperoxydase, la peroxydase de raifort et la chloroperoxydase.

Dans un mode de mise en oeuvre particulier du procédé de l'invention, le générateur de peroxyde est un générateur d'eau oxygénée; il peut en particulier être constitué par le couple glucose-glucose oxydase. Il est également possible de réaliser la radioiodation en utilisant directement le peroxyde d'hydrogène ($H_2O_2$).

Selon une autre caractéristique de l'invention, l'un des deux réactifs du couple glucoseglucose oxydase, en particulier le glucose, est ajouté en dernier lieu au milieu réactionnel comme initiateur de la réaction.

Il convient de noter qu'en variante, le générateur de peroxyde peut également être constitué par un persel, notamment un persulfate, par exemple le persulfate d'ammonium [$(NH_4)_2$ $S_2O_8$].

Selon un mode de mise en oeuvre particulier du procédé de l'invention, on réalise les diverses étapes successives suivantes:

- solubilisation des gangliosides dans un solvant organique, en particulier le mélange chloroformeméthanol (21);
- évaporation du solvant organique;
- addition au résidu d'évaporation d'un tampon phosphate;
- addition de Na. $^{125}I$ dissous dans un tampon phosphate;
- addition de la peroxydase préparée dans un tampon phosphate;
- addition de glucose-oxydase dissoute dans un tampon phosphate;
- addition de D-glucose dissous dans un tampon phosphate;
- évaporation du milieu réactionnel sous atmosphère d'azote à température ambiante, et
- séparation chromatographique.

Il convient ici de noter que la solubilisation du ou des gangliosides au sein de solvants organiques n'est toutefois pas

nécessaire. En effet, il est également possible de faire appel à des gangliosides qui sont présentés sous une forme lyophilisée et qui sont directement solubles dans le milieu réactionnel. Ils peuvent donc être radioiodés sans solubilisation préalable dans des solvants organiques ou des stabilisateurs.

D'autres caractéristiques et avantages de la présente invention apparaîtront à la lecture de la description détaillée faite ci-après en s'appuyant sur des exemples de mise en oeuvre particuliers.

**EXEMPLE 1: Radioiodation catalysée par la lactoperoxydase**

A) **Matériel utilisé**

Gangliosides: mélange purifié à partir de cerveau de boeuf (GM1 20 %, GD3 5 %, GD1a 40 %, GD1b 15 %, GT1b 17 %, GQ1 3 %, FIDIA, Abano Terme, Italie)
D-Glucose (Merck, RFA)
$^{125}I$ 55,5 x $10^4$ Bq/µg (15 mCi/µg $^{125}I$) (Amersham, GB)
Lactoperoxydase (EC 1. 11. 1. 7; 60 UI/mg protéine) (Sigma)
Glucose oxydase (EC 1. 1. 3. 4; 300 UI/mg protéine; d'origine Asperillus niger) (Sigma)
Neuraminidase (EC 3. 1. 1. 4 ; 150 UI/mg protéine d'origine Vibrio cholerae) (Sigma)
Tampons: phosphates (50 mM ou 100 mM à pH 6,0 ou 7,0)
Solvants organiques: de qualité Analar
Films, révélateurs et fixateurs: Agfa-Gevaert.
Cassettes pour autoradiographie: Phillips avec écran
Détecteur de radioactivité sur couche mince: Berthold, RFA
Silice H60: Merck. Gel pour chromatographie en milieu hydrophobe: LH-20 (Pharmacia, Suède)

B) **Mode opératoire de la réaction de radioiodation**

- Les gangliosides sont solubilisés dans un solvant organique, en particulier dans le mélange chloroforme: méthanol 2 : 1
- Evaporation des solvants organiques
- Addition de tampon phosphate (200 µl)
- Addition de 100 µCi de Na. $^{125}I$ (2,2 x $10^6$cpm) dans 10 µl de tampon phosphate

Na.$^{125}I$ + KI 1 mM ⊕ ⊕ = présence
Na.$^{125}I$ + KI 1 mM ⊖ ⊖ = absence

- Addition de 10 µg de LPO préparée dans du tampon phosphate, 10 µl

Na. $^{125}I$ + LPO + KI 1 mM ⊕
Na. $^{125}I$ + LPO + KI 1 mM ⊖

- Addition de glucose oxydase: 10 mU dans 10 µl de tampon phosphate

Na. $^{125}I$ + LPO + GO + KI 1 mM ⊕
Na. $^{125}I$ + LPO + GO + KI 1 mM ⊖

- Addition de D-Glucose: 250 nmoles dans 50 µl de tampon phosphate

Na.$^{125}I$ + LPO + GO + D-Glucose

La réaction est initiée par addition du glucose

D-Glucose + GO ⟶ $H_2O_2$ + Gluconolactone

$H_2O_2$ + $^{125}I$ + LPO + Gangliosides ⟶ (⅄ Gangliosides) + $^{125}I$ + $H_2O$ + LPO

La réaction dure 10 minutes à 20°C.

### C) Purification et chromatographie sur couche mince

Aucun réducteur n'est ajouté à la fin de la réaction (afin d'éviter la formation de sels de Bunte) et le milieu réactionnel est évaporé sous atmosphère d'azote à la température ambiante.

Le résidu est appliqué sur une colonne de silice (volume du gel 500 µl), préparé dans du chloroforme: méthanol: eau (74 : 21 : 1) ($E_1$), le premier éluat ($E_1$, 10 ml) est écarté et le deuxième (chloroforme: méthanol: eau (50 : 50 : 15) récupéré et évaporé dans un Rotovapor (Buchi, Suisse).

Le résidu est récupéré dans 20 µl de chloroforme-méthanol (2 : 1) et déposé sur couche mince de silice. Le dépôt est élué avec trois solvants différents selon la méthodede Dreyfus et coll. (HARTH, S.E., DREYFUS, H., URBAN, P.F. and MANDEL, P. (1978) Anal. Biochem. 86, 543):

- chloroforme: 1 heure
- chloroforme: méthanol: eau (70 : 30 : 4): 2 heures
- chloroforme: méthanol: eau (KCl 0,25 %) (60 : 35 : 8): 3 heures

Les plaques sont séchées, les produits marqués détectés par "scan" ou autoradiographie et ensuite révélés selon la méthode de Svennerholm et coll. (SVENNERHOLM, L., (1957) Biochim. Biophys. Acta 24, 604).

### D) Résultats

*** Radiochromatogramme**

Le radiochromatogramme des gangliosides radioiodés en l'absence d'iodure de potassium (Fig. 1) montre un fort marquage des espèces GT1.b, GD2 (a, b) et GM1.

De même, le radiochromatogramme des gangliosides radioiodés en présence d'iodure de potassium (Fig. 2) montre aussi le marquage des espèces GT1b, GD1 (a, b) et GM1.

Le traitement des gangliosides marqués à la neuraminidase (10 mU, incubation à 37°C pendant 30 minutes dans du tampon Tris-HCl, 50 mM, pH 7,2, contenant 100 µM de $CaCl_2$) montre que les produits marqués sont bien des gangliosides, car ils sont transformés en GM1 (Fig. 3).

*** Autoradiographie**

La révélation des produits radioactifs par scanning n'étant pas assez fine, vu la résolution basse du scanner, il a été procédé à l'autoradiographie (Fig. 4).

La piste 1 correspond à la figure 1, la piste 2 à la figure 2, et les pistes 3 et 4 correspondent aux gangliosides marqués sans (piste 3) ou avec (piste 4) présence d'iodure de potassium (1 mM) et ensuite traités à la neuraminidase.

L'autoradiographie montre clairement que les gangliosides, GT1b, GD1 (a, b) et GM1, sont radioiodés et que le traitement à la neuraminidase transforme ces derniers en GM1.

Il convient ici de bien noter que l'omission de LPO ou de glucose oxydase ne permet pas le marquage des gangliosides et seules des taches radioactives qui correspondent à $^{125}I_3^-$ et $^{125}I_2$ peuvent être détectées par autoradiographie.

Il a également été observé que l'activité spécifique est de $10^6$ cpm/ 5 µg d'acide N-acétyl neuraminique ou de 2 x $10^5$ cpm pour les gangliosides radioiodés en absence ou en présence de KI (1 mM).

Ainsi, il apparaît clairement que la LPO catalyse la radioiodation des gangliosides. Les résultats obtenus (marquage du GM1 et transformation du GT1b et des GD1 (a, b) en GM1 par la neuraminidase) confirme que la radioiodation se fait par substitution sur les chaînes paraffiniques composant le céramide.

Malgré la demi période (t $\frac{1}{2}$) de l'isotope relativement courte (61 jours), le procédé de l'invention permet d'obtenir des gangliosides par un élément à rayonnement γ, qui ne nécessitent donc pas de scintillateurs pour les détecter et peuvent donc être récupérés.

### EXEMPLE 2: Radioiodation d'un mélange de gangliosides catalysée par de la peroxydase de raifort insoluble

### A) Matériel utilise

a) Peroxydase insoluble (attachée à des billes d'agarose), (Sigma n° de catalogue P-3912, 23000 µg agarose ou 36 800 mU/100 µl de suspension)
b) gangliosides: mélange purifié à partir de cerveau de boeuf (GM1 20 %, GD3 5 %, GD1a 40 %, GD1b 15 %, GT1b 17 %, GQ1 3 %) (FIDIA, Abano Terme, Italie)
c) Glucose oxydase (300 UI/mg protéine d'origine Asperillus Niger), (Sigma, n° de catalogue G-6500)
d) D-Glucose, Merck

e) Tampon phosphate: 50 mM, pH = 7,0
f) KI: Prolabo
g) Chromatographie sur couche mince:
   1ère élution: $CHCl_3$, 1 heure
   2ème élution: $CHCl_3$, $CH_3OH$, $H_2O$ (70 : 30 : 4), 1 heure
   3ème élution: méthyl acétate: propanol 1: chloroforme: méthanol: eau (25 % KCl) (25 : 20 : 20 : 20: 17)

**B Mode opératoire**

- 100 µg de gangliosides contenus dans 10 µl de chloroforme: méthanol (2 : 1) sont déposés dans un tube à essai (Corning, borosilicate Glass, catalogue n° 99 445) et le solvant évaporé;
- 100 µl de tampon phosphate sont ajoutés;
- 100 µl de $^{125}I^-$ 3,7 x $10^6$ Bq (100 µCi) sont ajoutés (tampon phosphate;
- 100 µl de KI (1 mM tampon phosphate) sont ajoutés;
- 50 µl de suspension de peroxydase de raifort sont ajoutés (50 µl ont été prélevés du stock, rincés pendant 15 minutes avec le tampon phosphate (500 µl), centrifugés pendant 15 minutes à 4500 t/minute, et cette manipulation répétée encore deux fois, le culot a été resuspendu dans 50 µl de tampon phosphate);
- 70 mU de glucose oxydase dans 50 µl de tampon sont ajoutés;
- 50 µl de 0,09 % de glucose (tampon phosphate) sont ajoutés;
- La réaction dure 30 minutes et le tube est agité toutes les 5 minutes;
- Le milieu réactionnel est ensuite centrifugé pendant 30 minutes à 4500 t/minute, le surnageant récupéré, évaporé sous azote, le résidu repris dans 40 µl de chloroforme: méthanol (2 : 1) et déposé sur plaque de silice.

**C) Résultats**

La figure 5 (2 heures d'exposition du film à Rayon X après chromatographie) montre le marquage de GD1a, GD1b, GT1b et GQ1b (piste 2). L'omission de peroxydase de raifort ne permet pas le marquage des gangliosides.

**EXEMPLE 3: Radioiodation d'un mélange de gangliosides catalysée par de la lactoperoxydase**

**A) Matériel utilisé**

a) Lactoperoxydase (de lait, Sigma, Référence n° L-2005)
b) à f) comme à l'exemple 2
g) Chromatographie sur SEP-PAK® (phase inverse) ($C_{18}$ CARTRIDGE, WETERS Associates, millipore, Référence n° 51910)
h) Chromatographie sur couche mince: méthyl acétate: Propanol 1: chloroforme: methanol : eau (25 % KCl) (25 : 20 : 20 : 20 : 17) 1 heure

**B) Mode opératoire**

| | + lactoperoxydase | – lactoperoxydase | |
|---|---|---|---|
| gangliosides | 9 µg | 9 µg | |
| $^{125}I$ | 3,7 x $10^6$ Bq (100 µCi) | 3,7 x $10^6$ Bq (100 µCi) | dans 100 µl tampon phosphate |
| KI | 100 µl | 100 µ | 1 mM |
| lactoperoxydase | 50 µg | – | 50 µl dans tampon phosphate |
| glucose oxydase | 700 mU | 700 mU | |
| tampon phosphate | – | 50 µl | |
| glucose | 50 µl | 50 µl | (9 % dans $H_2O$) |

temps de réaction: 30 minutes

- Evaporation sous azote
- Le résidu est repris et mélangé dans 2 ml de chloroforme: méthanol: $H_2O$ (0,1 M KCl) (3 : 48 : 47) et appliqué sur la colonne SEP-PAK
- La colonne est éluée avec 3 ml du même solvant et ensuite avec 5 ml de ce solvant (cet éluat est mis de côté)
- La colonne est éluée avec 10 ml d'eau et l'éluat mis de côté
- La colonne est éluée avec 2 ml de méthanol et ensuite deux fois 2 ml de chloroforme: méthanol (1 : 1)

Ces éluats sont évaporés sous azote, resuspendus dans 500 µl de chloroforme: méthanol (2 : 1) et 50 µl déposés sur couche mince, (pour l'expérience où la lactoperoxydase est omise, le dépôt est de 100 µl).

### C) Résultats

La figure 6 (piste 1) montre le marquage des gangliosides par la lactoperoxydase et démontre l'élimination totale des espèces iodées ($I^-$, $I_2$, $I^-_3$, $I^-_5$,...) qui n'ont pas réagi.

Dans ces conditions, le rendement du marquage est de 100 % (1 atome d'iode par molécule de ganglioside) et le marquage suit la distribution des gangliosides marqués (GM1 20 %; GD3 3 %; GD1a 30 %; GD1b 15 %; GT1b 15 % et GQ1b 2 %; le GM3 se marque aussi 10 % et fait partie de la contamination du produit vendu par la FIDIA; le GM3 se marque fortement, car le petit copule glucidique favorise le marquage de la partie hydrophobe.

**EXEMPLE 4: Radioiodation d'un mélange de gangliosides catalysée par de la lactoperoxydase**

Le matériel utilise et le protocole opératoire sont identiques à ceux décrits dans le cadre de l'exemple 3, à l'exception toutefois du couple glucose-glucose oxydase qui a été remplacé par 50 µl d'une solution de persulfate d'ammonium à 11 %. Cette solution est ajoutée après l'addition de la lactoperoxydase.

Les gangliosides ont ainsi pu être radioiodés tel que cela apparaît sur la figure 7 (piste 1).

**EXEMPLE 5: Radioiodation d'un mélange de gangliosides catalysée par de la lactoperoxydase**

Le matériel utilisé et le protocole opératoire sont identiques à ceux décrits dans le cadre de l'exemple 3, à l'exception toutefois du couple glucose-glucose oxydase qui a été remplacé par 3 µl d'$H_2O_2$ à 30 % (Prolabo®). Cette solution est ajouté après l'addition de la lactoperoxydase.

Les gangliosides ont ainsi pu être radioiodés tel que cela apparaît sur la figure 7 (piste 2).

**EXEMPLE 6: Radioiodation d'un mélange de gangliosides catalysée par de la lactoperoxydase**

Le matériel utilisé et le protocole opératoire sont identiques à ceux décrits dans le cadre de l'exemple 3, à l'exception toutefois du couple glucose-glucose oxydase qui à été remplacé par dix additions successives de 3 µl d'$H_2O_2$ à 3 %, à des intervalles de 3 minutes. Cette solution est ajouté après l'addition de la lactoperoxydase.

Les gangliosides ont ainsi pu être radioiodés tel que cela apparaît sur la figure 7 (piste 3).

**EXEMPLE 7: Radioiodation d'un ganglioside (GM1) catalysée par la lactoperoxydase**

En faisant appel au matériel et au protocole opératoire utilisés dans le cadre de l'exemple 3, on a réalisé la radioiodation de 20 µg de GM1, tel que cela apparaît sur la figure 8 (piste 1). Il est en outre apparu que la neuraminidase ne dénaturait pas le produit radioiodé (Fig. 8, piste 2).

## Revendications

1. Procédé de radioiodation de gangliosides, caractérisé en ce que l'on met à réagir une solution aqueuse d'au moins un ganglioside avec un composé iodé radioactif, en particulier un iodure $^{125}I^-$, en présence d'une peroxydase catalysant les réactions d'halogénation et d'un peroxyde ou générateur de peroxyde, puis après achèvement de la réaction, en ce que l'on purifie et l'on récupère les gangliosides radiomarqués.

2. Procédé selon la revendication 1, caractérisé en ce que ladite peroxydase est choisie parmi la lactoperoxydase, la peroxydase de raifort et la chloroperoxydase.

3. Procédé selon l'une des revendications 1 et 2, caractérisé en ce que le générateur de peroxyde est un générateur d'eau oxygénée.

4. Procédé selon la revendication 3, caractérisé en ce que le générateur d'eau oxygénée est constitué par le couple glucose-glucose oxydase.

5. Procédé selon la revendication 4, caractérisé en ce que l'un des deux réactifs du couple glucose-glucose oxydase, en particulier le glucose, est ajouté en dernier lieu au milieu réactionnel comme initiateur de la réaction.

6. Procédé selon la revendication 1, caractérisé en ce que le générateur de peroxyde est constitué par un persel, tel que le persulfate d'ammonium [$(NH_4)_2 S_2O_8$].

7. Procédé selon la revendication 1, caractérisé en ce que le peroxyde utilisé est le peroxyde d'hydrogène ($H_2O_2$).

8. Procédé selon l'une des revendications 1 à 7, caractérisé en ce que les différents réactifs sont ajoutés au milieu réactionnel en présence d'un tampon phosphate.

9. Procédé selon l'une des revendications 1 à 8, caractérisé en ce que la température réactionnelle est voisine de la température ambiante.

10. Procédé selon l'une des revendications 1 à 9, caractérisé en ce que le ou les ganglioside(s) de départ est ou (sont) choisi(s) parmi GM3, GM2, GM1, GD3, GD1a, GD1b, GT1b, GQ, et toute molécule comprenant un groupement céramide et un copule glucidique possédant au moins un groupement acide N-acétyl-neuraminique, ainsi que leurs mélanges.

11. Procédé selon l'une des revendications 1 à 10, caractérise en ce que les gangliosides de départ sont constitués par un mélange purifié de gangliosides obtenus à partir de cerveau de boeuf, en particulier un mélange répondant à la composition suivante:

| | |
|---|---|
| GM1 | 20 % |
| GD3 | 5 % |
| GD1a | 40 % |
| GD1b | 15 % |
| GT1b | 17 % |
| GQ1 | 3 % |

12. Procédé selon l'une des revendications 1 à 11, caractérisé en ce que la radioiodation consiste en une substitution sur les chaînes paraffiniques de la fraction céramide des gangliosides.

13. Procédé selon l'une des revendications 1 à 12, caractérisé en ce que l'étape de purification est obtenue par évaporation du milieu réactionnel sous atmosphère d'azote à température ambiante, suivie d'une séparation chromatographique.

14. Procédé selon l'une des revendications 1 à 13, caractérisé par la mise en oeuvre des étapes successives suivantes:

- solubilisation des gangliosides dans un solvant organique, en particulier le mélange chloroformeméthanol (2 - 1);
- évaporation du solvant organique;
- addition au résidu d'évaporation d'un tampon phosphate;
- addition de Na. $^{125}I$ dissous dans un tampon phosphate;
- addition de la peroxydase préparée dans un tampon phosphate;
- addition de glucoseoxydase dissoute dans un tampon phosphate;
- addition de D-glucose dissous dans un tampon phosphate;
- évaporation du milieu réactionnel sous atmosphère d'azote à température ambiante, et
- séparation chromatographique.

**Claims**

1. Method for the radioiodination of gangliosides, characterized in that an aqueous solution of at least one ganglioside is reacted with a radioactive iodinated compound, especially a $^{125}I^-$ iodide, in the presence of a peroxidase that catalyses halogenation reactions and of a peroxide or peroxide generator, and then, after completion of the reaction, in that the radiolabelled gangliosides are purified and recovered.

2. Method according to Claim 1, characterized in that the said peroxidase is chosen from lactoperoxidase, horseradish peroxidase and chloroperoxidase.

3. Method according to one of Claims 1 and 2, characterized in that the peroxide generator is a hydrogen peroxide generator.

4. Method according to Claim 3, characterized in that the hydrogen peroxide generator consists of the glucose/glucose oxidase system.

5. Method according to Claim 4, characterized in that one of the two reactants of the glucose/glucose oxidase system, especially the glucose, is added last to the reaction medium as a reaction initiator.

6. Method according to Claim 1, characterized in that the peroxide generator consists of a persalt, such as ammonium persulphate [$(NH_4)_2S_2O_8$].

7. Method according to Claim 1, characterized in that the peroxide used is hydrogen peroxide ($H_2O_2$).

8. Process according to one of Claims 1 to 7, characterized in that the different reactants are added to the reaction medium in the presence of a phosphate buffer.

9. Method according to one of Claims 1 to 8, characterized in that the reaction temperature is in the region of room temperature.

10. Method according to one of Claims 1 to 9, characterized in that the starting ganglioside or gangliosides is/are chosen from GM3, GM2, GM1, GD3, GD1a, GD1b, GT1b, GQ and any molecule containing a ceramide group and a sugar moiety possessing at least one N-acetylneuraminic acid group, as well as the mixtures thereof.

11. Method according to one of Claims 1 to 10, characterized in that the starting gangliosides consist of a purified mixture of gangliosides obtained from ox brain, especially a mixture corresponding to the following composition:

| | |
|---|---|
| GM1 | 20 % |
| GD3 | 5 % |
| GD1a | 40 % |
| GD1b | 15 % |
| GT1b | 17 % |
| GO1 | 3 % |

12. Method according to one of Claims 1 to 11, characterized in that the radioiodination consists of a substitution on the paraffin chains of the ceramide fraction of the gangliosides.

13. Method according to one of Claims 1 to 12, characterized in that the purification stage is obtained by evaporation of the reaction medium under a nitrogen atmosphere at room temperature, followed by a chromatographic separation.

14. Method according to one of Claims 1 to 13, characterized in that the following successive stages are carried out:

- solubilization of the gangliosides in an organic solvent, in particular a chloroform/methanol (2 : 1) mixture;
- evaporation of the organic solvent;
- addition of a phosphate buffer to the evaporation residue;
- addition of Na $^{125}I$ dissolved in a phosphate buffer;
- addition of the peroxidase prepared in a phosphate buffer;
- addition of glucose oxidase dissolved in a phosphate buffer;
- addition of D-glucose dissolved in a phosphate buffer;
- evaporation of the reaction medium under a nitrogen atmosphere at room temperature; and
- chromatographic separation.

**Patentansprüche**

1. Verfahren zur Radiojodierung von Gangliosiden, *dadurch gekennzeichnet*, daß man eine wäßrige Lösung mindestens eines Gangliosids mit einer radioaktiven jodhaltigen Verbindung, insbesondere einem Jodid $^{125}I^-$, in Anwesenheit einer Peroxidase, die die Halogenierungsreaktionen katalysiert, und eines Peroxids oder Peroxiderzeugers reagieren läßt, und nach vollendeter Reaktion die radiomarkierten Ganglioside reinigt und gewinnt.

2. Verfahren nach Anspruch 1, *dadurch gekennzeichnet*, daß die Peroxidase ausgewählt wird aus Lactoperoxidase, Meerrettich-Peroxidase und Chlorperoxidase.

3. Verfahren nach einem der Ansprüche 1 und 2, *dadurch gekennzeichnet*, daß der Peroxiderzeuger ein Erzeuger von Wasserstoffperoxid ist.

4. Verfahren nach Anspruch 3, *dadurch gekennzeichnet*, daß der Wasserstoffperoxid-Erzeuger aus dem Glucose-Glucoseoxidase-Paar besteht.

5. Verfahren nach Anspruch 4, *dadurch gekennzeichnet*, daß die beiden Reaktionskomponenten des Glucose-Glucoseoxidase-Paares, insbesondere die Glucose, dem Reaktionsmedium als Reaktionsinitiator zuletzt zugesetzt werden.

6. Verfahren nach Anspruch 1, *dadurch gekennzeichnet*, daß der Peroxiderzeuger aus einem Persalz, wie dem Ammoniumpersulfat [$(NH_4)_2$ $S_2O_8$], besteht.

7. Verfahren nach Anspruch 1, *dadurch gekennzeichnet*, daß das verwendete Peroxid Wasserstoffperoxid ($H_2O_2$) ist.

8. Verfahren nach einem der Ansprüche 1 bis 7, *dadurch gekennzeichnet*, daß die verschiedenen Reagenzien dem Reaktionsmedium in Anwesenheit eines Phosphatpuffers zugesetzt werden.

9. Verfahren nach einem der Ansprüche 1 bis 8, *dadurch gekennzeichnet*, daß die Reaktionstemperatur in der Gegend der Raumtemperatur liegt.

10. Verfahren nach einem der Ansprüche 1 bis 9, *dadurch gekennzeichnet*, daß das oder die Ausgangs-Gangliosid(e) ausgewählt wird (werden) aus GM3, GM2, GM1, GD3, GD1a, GD1b, GT1b, GQ und dem ganzen Molekül, das eine Ceramidgruppe und eine glucidische Verknüpfung umfaßt, die mindestens eine N-Acetylneuraminsäure-Gruppe besitzt, sowie deren Gemischen.

11. Verfahren nach einem der Ansprüche 1 bis 10, *dadurch gekennzeichnet*, daß die Ausgangsganglioside aus einem gereinigten Gemisch von Gangliosiden gebildet werden, die aus Rinderhirn erhalten wurden, insbesondere einem Gemisch, das der folgenden Zusammensetzung entspricht:

| | |
|---|---|
| GM1 | 20 % |
| GD3 | 5 % |
| GD1a | 40 % |
| GD1b | 15 % |
| GT1b | 17 % |
| GQ1 | 3 % |

12. Verfahren nach einem der Ansprüche 1 bis 11, *dadurch gekennzeichnet*, daß die Radiojodierung in einer Substitution an den paraffinischen Ketten der Ceramidfraktion der Ganglioside besteht.

13. Verfahren nach einem der Ansprüche 1 bis 12, *dadurch gekennzeichnet*, daß die Reinigungsstufe durch Verdampfen des Reaktionsmediums unter Stickstoffatmosphäre bei Raumtemperatur, gefolgt von einer chromatographischen Trennung, erfolgt.

14. Verfahren nach einem der Ansprüche 1 bis 13, *dadurch gekennzeichnet*, daß folgende Stufen nacheinander durchgeführt werden:

- Lösen der Ganglioside in einem organischen Lösungsmittel, insbesondere dem Gemisch Chloroform-Methanol (2 - 1);
- Verdampfen des organischen Lösungsmittels;
- Zusatz eines Phosphatpuffers zu dem Verdampfungsrückstand;
- Zusatz von Na. $^{125}I$, gelöst in einem Phosphatpuffer;
- Zusatz der in einem Phosphatpuffer bereiteten Peroxidase;
- Zusatz der Glucoseoxidase, gelöst in einem Phosphatpuffer;
- Zusatz der D-Glucose, gelöst in einem Phosphatpuffer;
- Verdampfen des Reaktionsmediums unter Stickstoffatmosphäre bei Raumtemperatur, und
- chromatographische Trennung.

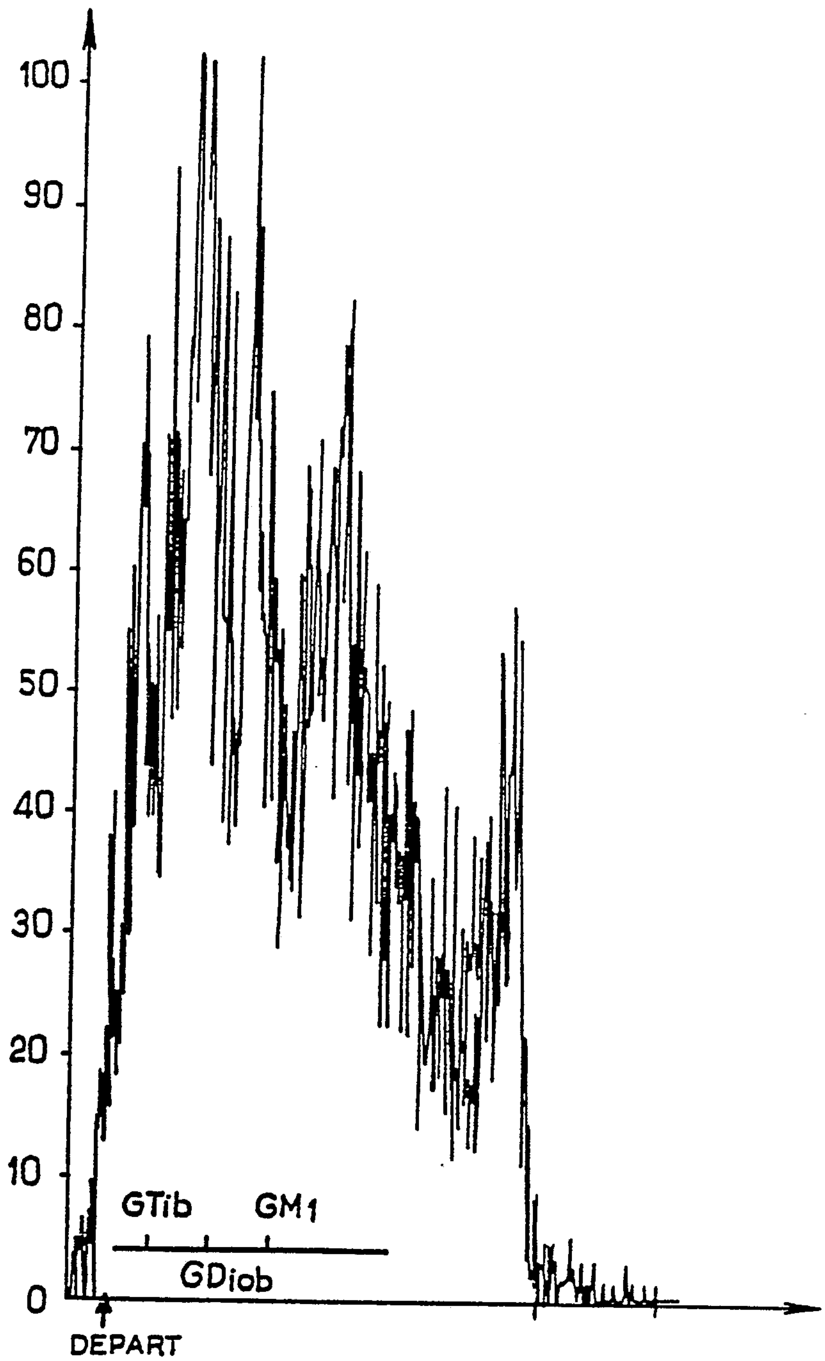


FIG_1

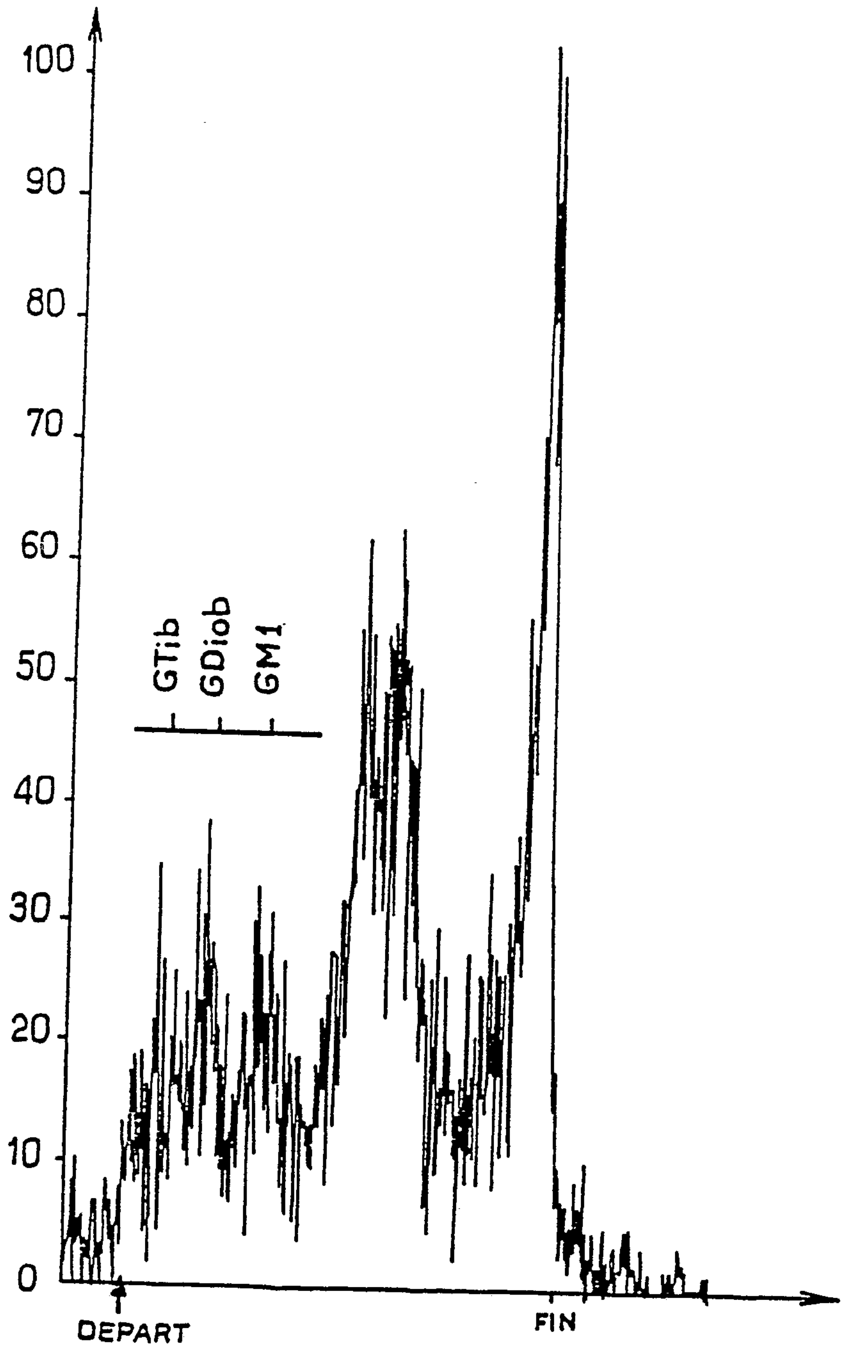


FIG_2

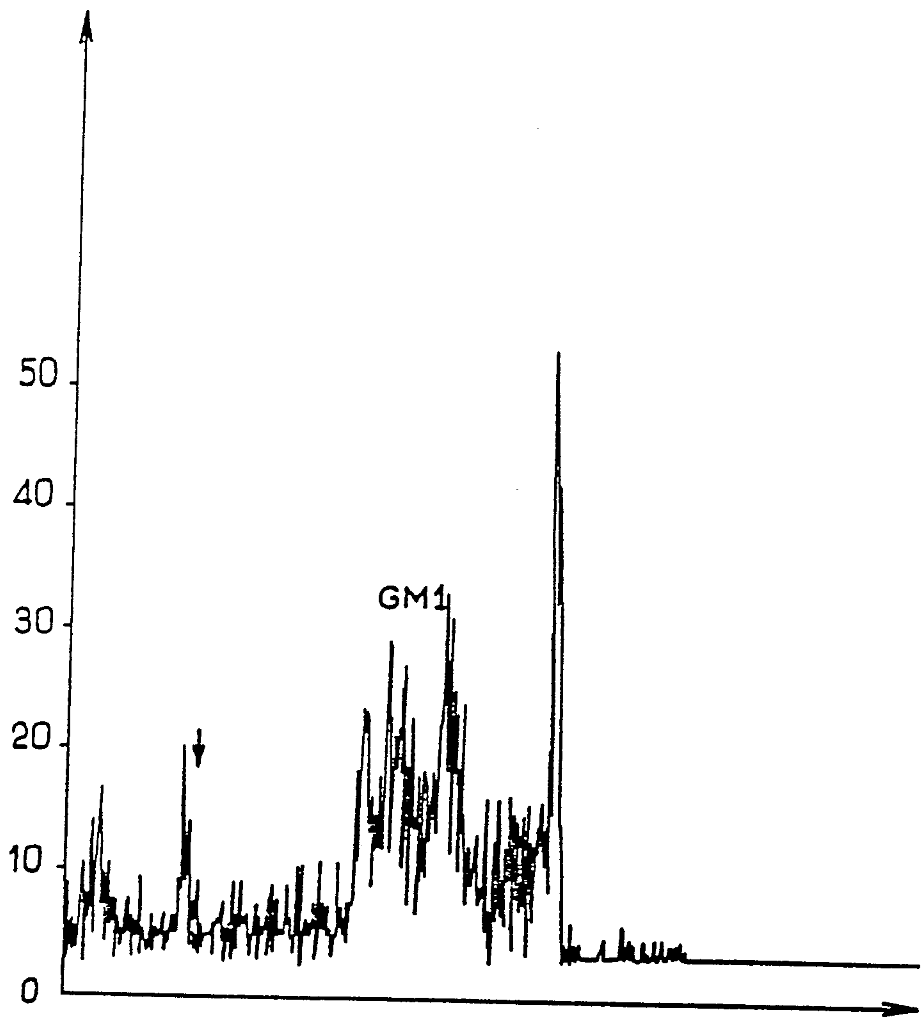


FIG_3

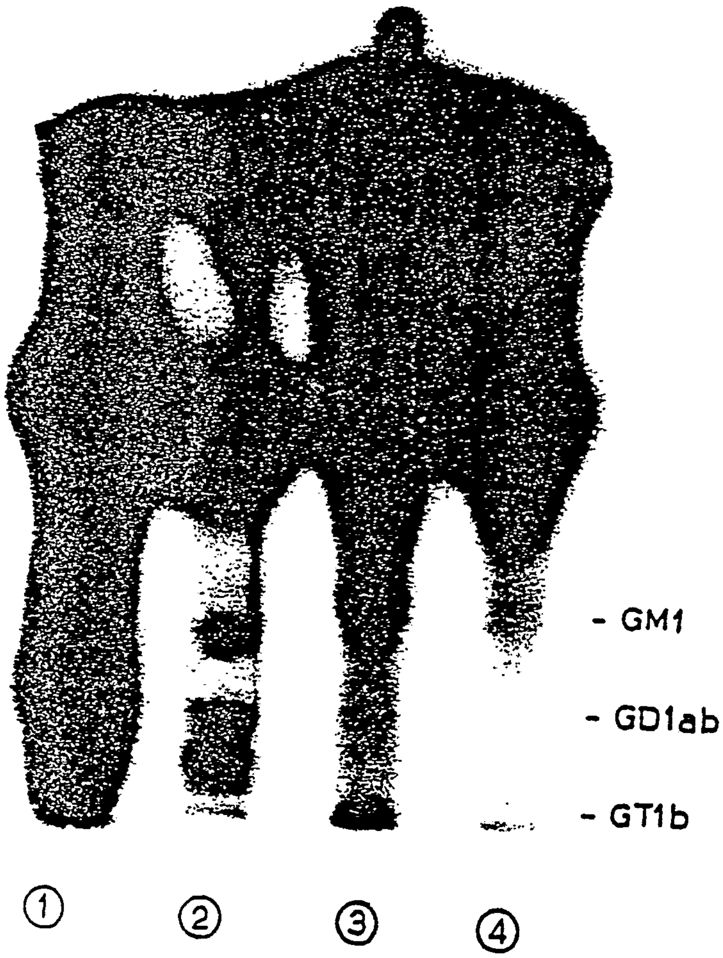


FIG_4

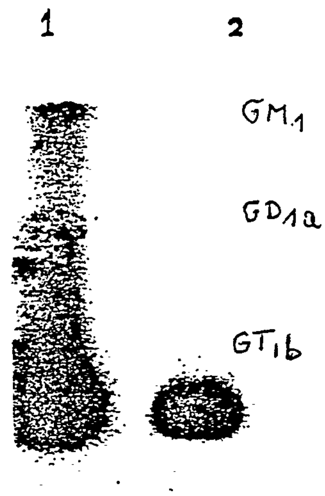


FIG_5

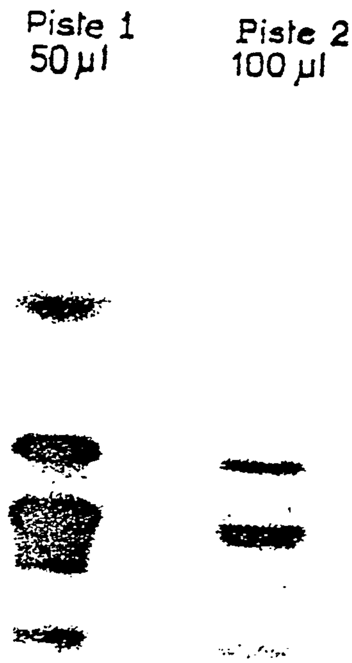


FIG. 6

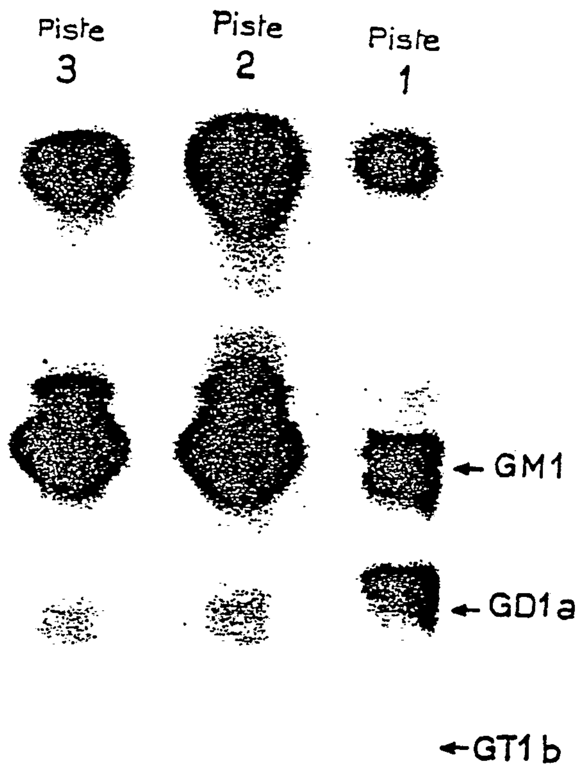


FIG_7

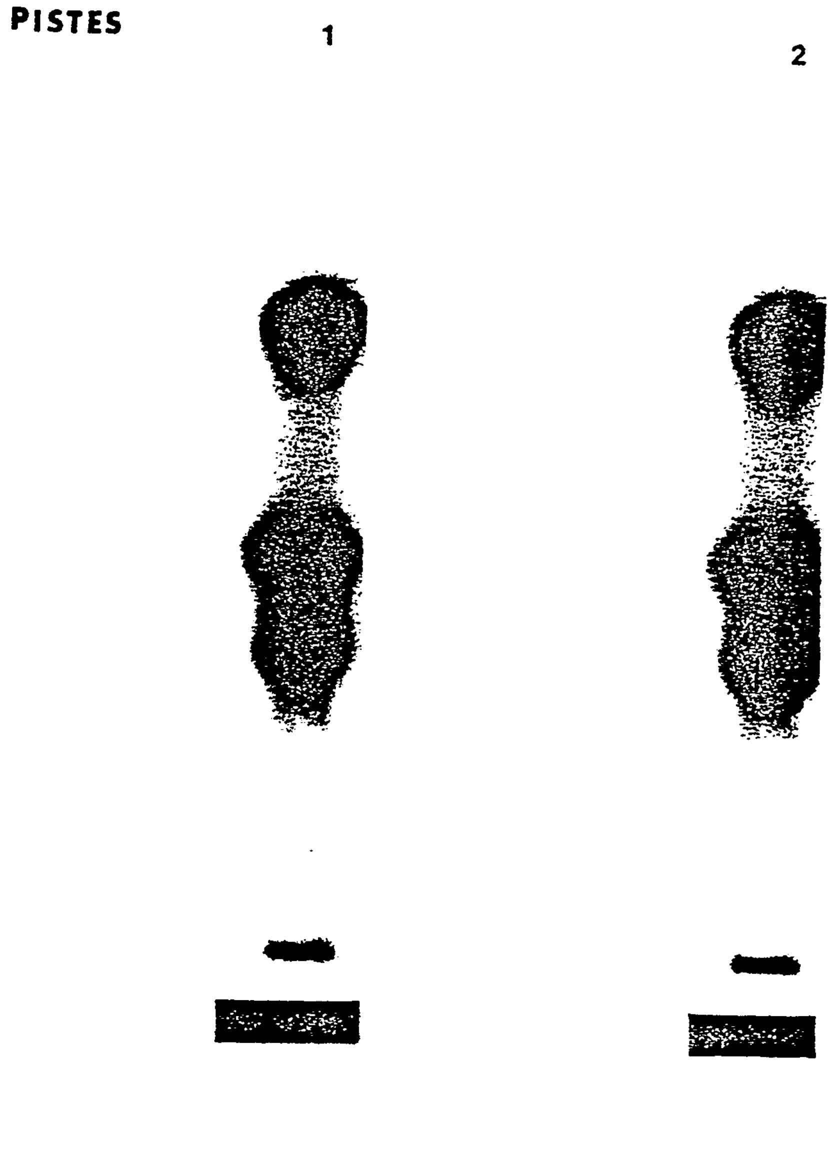


FIG_8